# EUROPEAN PATENT APPLICATION

(11) **EP 4 692 066 A1**
(43) Date of publication of application: **11.02.2026**
(21) Application number: 24777997.8
(22) Date of filing: 26.03.2024
(51) Int. Cl.: C07D 237/26, C07B 41/04, C07B 63/00

(54) **METHOD FOR SYNTHESIZING KEY INTERMEDIATE OF SELECTIVE THR BETA AGONIST**

(30) Priority: 28.03.2023 CN 202310310493
(71) Applicant: Haisco Pharmaceutical Group Co., Ltd., Shannan, Tibet 856000 (CN)
(72) Inventor: FAN, Jiang, Lhoka, Tibet 856099 (CN); WANG, Zhigang, Lhoka, Tibet 856099 (CN); LIU, Chang, Lhoka, Tibet 856099 (CN)
(74) Representative: AWA Denmark A/S
(86) International application number: PCT/CN2024/083764
(87) International publication number: WO 2024/199209

(57) **Abstract**

The present invention provides a method for synthesizing a key intermediate compound represented by formula (II) of a selective THR beta agonist. Raw materials used in the method are inexpensive and readily available, the process is simple and efficient, no SFC chiral separation is required, the yield is high, quality and costs are controllable, and the method has great industrial prospects.

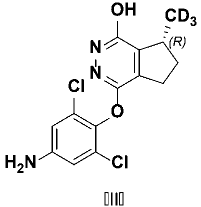

## Description

### Technical Field

The present invention relates to a method for preparing a pharmaceutical compound, and in particular to a method for preparing a selective THR beta agonist and a key intermediate thereof, which belongs to the technical field of pharmaceutical chemistry.

### Background Art

Classical familial hypercholesterolemia (FH) is an autosomal (co) dominant genetic disorder, with the main clinical manifestation of a marked increase in serum low density lipoprotein-cholesterol (LDL-C) level, as well as skin/tendon xanthoma, and can lead to early cardiovascular disease. According to statistics, the prevalence of HeFH is as high as 0.2%-0.48%. FH is usually caused by inactivating mutations in the LDL receptor (LDLR). In clinical use, statins are the drugs of first choice for treatment, not only to reduce LDL-C levels, but also to improve the prognosis of the patients with FH. Patients who do not respond well to statin therapy or have adverse reactions can be treated in combination with the cholesterol absorption inhibitor, ezetimibe. PCSK9 inhibitor may be additionally administered to patients who do not reach the standard cholesterol level after the treatments described above. Although there are corresponding treatments with respect to HeFH, many patients (up to 40% of HeFH patients) are unable to reach the standard level of cholesterol (LDL-C) after these treatments, and the accumulation of cholesterol in their bodies becomes a lifelong burden. Furthermore, studies have shown that binding of thyroid hormone to the beta subtype thyroid hormone receptor can promote the cholesterol metabolism. As a result, the development of thyroid hormone analogues or thyromimetics that selectively agonize the β subtype receptor may be used for treating FH.

In the compound patent WO 2019240938 A1, a compound represented by formula (I), which is a thyroxine beta receptor agonist and is intended for the treatment of primary hypercholesterolemia of adult, is described and a preparation method therefor is also disclosed. However, the preparation method has problems such as poor intermediate 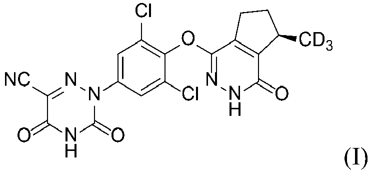 stability and difficult purification, and has the disadvantages of requiring multiple steps of column purification which is not conducive to industrial application, reaction conditions that are not conducive to large-scale production, low yield, and high cost. Furthermore, the preparation of the intermediate represented by formula (II) in the preparation method is highly dependent on chiral separation methods such as SFC. Moreover, the intermediate is difficult to separate, and its supply is restricted by the production capacity of chiral separation. The current supply market cannot meet the commercial production needs.

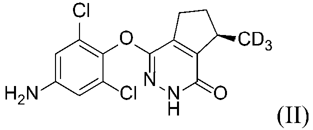

Based on the above background, the present invention proposes a new synthetic route for intermediate (II), which has advantages of easy scale-up production, controllable quality and controllable cost. Compared with the original route, the new route does not require chiral separation methods such as SFC, and the yield is significantly improved. Furthermore, the present invention further proposes a synthesis process for the starting material (formula III) of the new route, which route has inexpensive and readily available raw materials, simple and efficient process, and great industrial prospects.

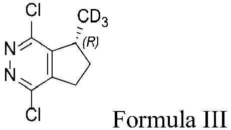

### Summary of the Invention

The present invention provides a method for preparing an intermediate represented by formula (II), the method comprising the following process route:

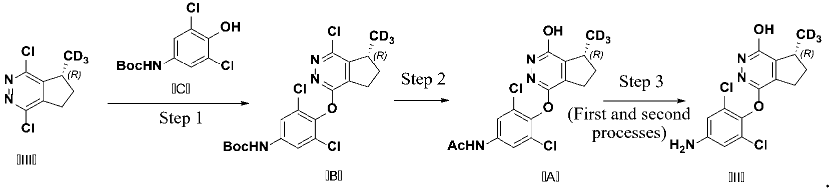

In some embodiments, the step 1 comprises: coupling an intermediate represented by formula (III) with a compound represented by formula (C) under nitrogen atmosphere in the presence of a palladium catalyst, a ligand, a base, and a solvent under heating or at room temperature to obtain a compound represented by formula (B).

In some embodiments, the palladium catalyst in the step 1 is selected from at least one of palladium acetate (CAS: 3375-31-3), palladium dichloride (CAS: 7647-10-1), tetrakis(triphenylphosphine)palladium (CAS: 14221-01-3), allylpalladium chloride dimer (CAS: 12012-95-2), bis(2-methylallyl)palladium chloride (CAS: 12081-18-4), (2-butenyl)chloropalladium dimer (12081-22-0), bis(acetonitrile)palladium dichloride (CAS: 14592-56-4), tris(dibenzylideneacetone)dipalladium (CAS: 52409-22-0), bis(dibenzylideneacetone)palladium (CAS: 32005-36-0), palladium trifluoroacetate (CAS: 42196-31-6) and palladium hydroxide (CAS: 12135-22-7).

In some embodiments, the ligand in the step 1 is selected from at least one of a monophosphorus ligand or a diphosphorus ligand; the monophosphorus ligand is selected from at least one of triphenylphosphine, tributylphosphine, tricyclohexylphosphine, diadamantylbutylphosphine, X-Phos (CAS: 564483-18-7), RuPhos (CAS: 787618-22-8) and tri(2-furyl)phosphine; and the diphosphorus ligand is selected from at least one of dppf, dppp, dppb, dppbz, dtbpf, dppNap, Xant-phos, an oxygen-phosphorus ligand, a nitrogen-phosphorus ligand, a nitrogen-nitrogen ligand and a nitrogen-oxygen ligand.

In some embodiments, the base in the step 1 is selected from at least one of lithium carbonate, sodium carbonate, potassium carbonate, caesium carbonate, sodium bicarbonate, potassium bicarbonate, lithium acetate, sodium acetate, potassium acetate, caesium acetate, triethylamine and diisopropylethylamine.

In some embodiments, the solvent in the step 1 is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

In some embodiments, the reaction in the step 2 is carried out under alkaline conditions, and the base used is selected from at least one of lithium acetate, sodium acetate, potassium acetate, caesium acetate, potassium phosphate, sodium phosphate, disodium phosphate, dipotassium phosphate, sodium hydroxide, lithium hydroxide, potassium hydroxide, calcium hydroxide, caesium hydroxide (hydrate), lithium carbonate, sodium carbonate, potassium carbonate, caesium carbonate, calcium carbonate, sodium bicarbonate, potassium bicarbonate and lithium bicarbonate; and the reaction solvent is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

In some embodiments, the step 3 comprises a first process and a second process, the first process is carried out under alkaline conditions, and the base used is selected from at least one of lithium hydroxide, sodium hydroxide, potassium hydroxide, caesium hydroxide and a hydrate thereof, lithium carbonate, sodium carbonate, potassium carbonate, caesium carbonate, lithium acetate, sodium acetate, potassium acetate and caesium acetate; the second process is carried out under acidic conditions, and the acid used is selected from at least one of hydrochloric acid, sulfuric acid, nitric acid, formic acid, glacial acetic acid, methanesulfonic acid, p-toluenesulfonic acid and a hydrate thereof, citric acid, oxalic acid, ammonium bisulphate and sodium bisulphate; and the reaction solvent used in the step 3 is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

Further, the method for preparing the intermediate represented by formula (II) of the present invention further comprises the following purification step: 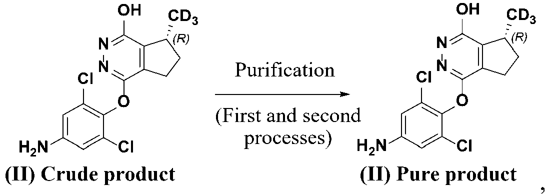 in some embodiments, the first process in the purification step described above is carried out under alkaline conditions, and the base used is selected from at least one of lithium hydroxide, sodium hydroxide, potassium hydroxide, caesium hydroxide and a hydrate thereof, lithium carbonate, sodium carbonate, potassium carbonate, caesium carbonate, lithium acetate, sodium acetate, potassium acetate and caesium acetate; the second process is carried out under acidic conditions, and the acid used is selected from at least one of hydrochloric acid, sulfuric acid, nitric acid, formic acid, glacial acetic acid, methanesulfonic acid, p-toluenesulfonic acid and a hydrate thereof, citric acid, oxalic acid, ammonium bisulphate and sodium bisulphate; and the solvent used in the purification step is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

The present invention further provides a method for preparing an intermediate represented by formula (III), the method comprising the following process route:

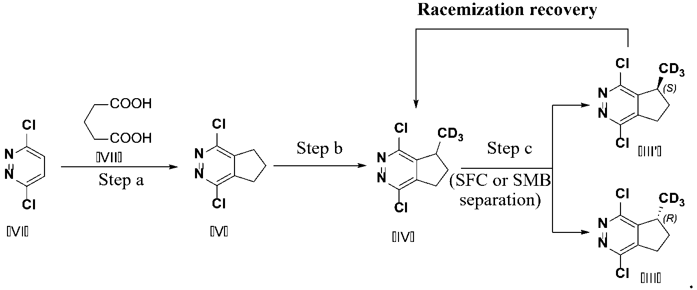

In some embodiments, the step a comprises: subjecting a compound represented by formula (VI) and a compound represented by formula (VII) to a Minisci reaction in the presence of a silver salt, persulphate and a solvent to obtain a compound represented by formula (V); the silver salt is selected from at least one of silver carbonate, silver nitrate, silver iodide, silver bromide, silver chloride and silver sulphate; the persulphate is selected from at least one of ammonium sulphate, magnesium sulphate, sodium sulphate, potassium sulphate, calcium sulphate, iron sulphate, copper sulphate, potassium sulphate and aluminium sulphate; and the solvent is selected from at least one of dichloromethane, trichloromethane, purified water, N,N-dimethylacetamide, N,N-dimethylformamide, N-methylpyrrolidin-2-one, dimethylsulfoxide, sulfolane, n-hexane, n-heptane, glacial acetic acid, methanol, ethanol, isopropanol, acetonitrile, propanenitrile, tetrahydrofuran and 2-methyltetrahydrofuran.

In some embodiments, the step b comprises: subjecting the compound represented by formula (V), after being treated with a base, to a substitution reaction with a deuterated methylating reagent to obtain a compound represented by formula (IV); the base is selected from at least one of lithium diisopropylamide, lithium hexamethyldisilazide, caesium carbonate, potassium carbonate, potassium tert-butoxide, sodium tert-butoxide, n-butyllithium and tert-butyllithium; and the deuterated methylation reagent is selected from at least one of deuterated iodomethane, deuterated dimethyl sulphate, deuterated methyl trifluoromethanesulfonate and deuterated methyl p-toluenesulfonate.

In some embodiments, the racemization recovery comprises: subjecting a compound represented by formula (III') to a racemization reaction in the presence of a base and a solvent to obtain the compound represented by formula (IV); the base is selected from at least one of potassium tert-butoxide, sodium tert-butoxide, tert-butyllithium, n-butyllithium, sodium ethoxide, sodium methoxide, caesium carbonate, potassium carbonate, sodium carbonate, lithium diisopropylamide and lithium hexamethyldisilazide; and the solvent is selected from at least one of methanol, ethanol, isopropanol, ethyl acetate, isopropyl acetate, acetic acid, formic acid, methyl tert-butyl ether, acetonitrile, propanenitrile, n-butanol, dimethylsulfoxide, sulfolane, N,N-dimethylacetamide, N,N-dimethylformamide, tetrahydrofuran, 2-methyltetrahydrofuran, acetone, butanone, cyclohexane, n-heptane, benzene and toluene.

In addition, the present invention further provides a compound represented by formula (B):

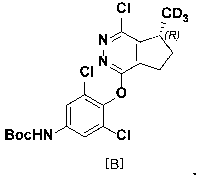

Moreover, the present invention further provides a method for preparing the compound represented by formula (B), the method comprising the following step 1: 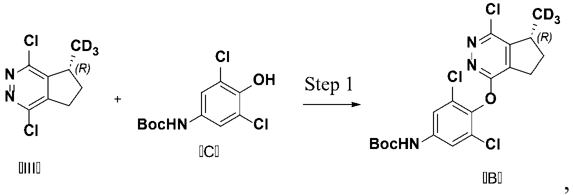 wherein the step 1 comprises: coupling the intermediate represented by formula (III) with the compound represented by formula (C) under nitrogen atmosphere in the presence of a palladium catalyst, a ligand, a base, and a solvent under heating or at room temperature to obtain the compound represented by formula (B);
in some embodiments, the palladium catalyst is selected from at least one of palladium acetate, palladium dichloride, tetrakis(triphenylphosphine)palladium, allylpalladium chloride dimer, bis(2-methylallyl)palladium chloride, (2-butenyl)chloropalladium dimer, bis(acetonitrile)palladium dichloride, tris(dibenzylideneacetone)dipalladium, bis(dibenzylideneacetone)palladium, palladium trifluoroacetate and palladium hydroxide;
in some embodiments, the ligand is selected from at least one of a monophosphorus ligand or a diphosphorus ligand; the monophosphorus ligand is selected from at least one of triphenylphosphine, tributylphosphine, tricyclohexylphosphine, diadamantylbutylphosphine, X-Phos, RuPhos and tri(2-furyl)phosphine; and the diphosphorus ligand is selected from at least one of dppf, dppp, dppb, dppbz, dtbpf, dppNap, Xant-phos, an oxygen-phosphorus ligand, a nitrogen-phosphorus ligand, a nitrogen-nitrogen ligand and a nitrogen-oxygen ligand;
in some embodiments, the base is selected from at least one of lithium carbonate, sodium carbonate, potassium carbonate, caesium carbonate, sodium bicarbonate, potassium bicarbonate, lithium acetate, sodium acetate, potassium acetate, caesium acetate, triethylamine and diisopropylethylamine;
in some embodiments, the solvent is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

Unless stated to the contrary, the terms used in the present application have the following meanings.

The salt of a compound refers to a salt obtained by reacting a free acid with an inorganic base or an organic base, or reacting a free base with an inorganic acid or an organic acid.

"Alcohol solvent" refers to a solvent containing hydroxyl in the molecular structure, and non-limiting examples include ethylene glycol, methanol, ethanol, n-propanol, isopropanol, n-butanol, n-pentanol, sec-pentanol, 3-pentanol, isopentanol, tert-pentanol, n-hexanol, cyclohexanol, etc.

"Ether solvent" refers to a solvent containing an ether bond in the molecular structure, and non-limiting examples include tetrahydrofuran, 2-methyltetrahydrofuran, diethyl ether, 1,4-dioxane, methyl tert-butyl ether, ethylene glycol dimethyl ether, diisopropyl ether, ethylbutyl ether, dibutyl ether, dipentyl ether, diethylene glycol dimethyl ether, triethylene glycol dimethyl ether, anisole, etc.

"Aromatic hydrocarbon solvent" refers to a solvent containing an aromatic ring having 0-3 heteroatoms (the heteroatoms are selected from O, S or N) in the molecular structure, and non-limiting examples include benzene, pyridine, toluene, ethylbenzene, xylene, chlorobenzene, o-dichlorobenzene, etc.

"Halogenated alkane solvent" refers to an alkane solvent containing halogen (fluorine, chlorine, bromine or iodine) in the molecular structure, and non-limiting examples include dichloromethane, 1,2-dichloroethane, chloroform, trichloroethane, carbon tetrachloride, pentachlorohexane, 1-chlorobutane, tribromomethane, etc.

"Alkane solvent" refers to a solvent containing only alkane in the molecular structure, and non-limiting examples include n-hexane, n-heptane, n-octane, n-pentane, cyclohexane, cycloheptane, etc.

"Ester solvent" refers to a solvent containing a carboxylic ester in the molecular structure, and non-limiting examples include ethyl acetate, isopropyl acetate, glyceryl triacetate, ethyl acetoacetate, isopentyl acetate, isopropyl acetate, n-butyl acetate, n-propyl acetate, n-pentyl acetate, methyl acetate, sec-butyl acetate, butyl formate, propyl formate, n-pentyl formate, diethyl carbonate, etc.

"Ketone solvent" refers to a solvent containing ketocarbonyl in the molecular structure, and non-limiting examples include acetone, butanone, acetophenone, methyl isobutyl ketone, 2,6-dimethyl-2,5-heptadien-4-one, 3,5,5-trimethyl-2-cyclohexenone, mesityl oxide, etc.

"Nitrile solvent" refers to a solvent containing cyano in the molecular structure, and non-limiting examples include acetonitrile, propionitrile, butyronitrile, phenylacetonitrile, etc.

"Amide solvent" refers to a solvent containing amide in the molecular structure, and non-limiting examples include N,N-dimethylformamide, N,N-dimethylacetamide, N,N-diethylacetamide, hexamethylphosphoramide, N-methylpyrrolidone, etc.

"Polar aprotic solvent" refers to a solvent that does not contain a hydrogen atom directly connected to an electronegative atom and has no hydrogen bonding ability. Non-limiting examples of polar aprotic solvents include acetone, dimethylsulfoxide, HMF (hydroxymethylfurfural), crown ether, acetonitrile, N,N-dimethylformamide, N,N-diethylformamide, N,N-dimethylacetamide, dimethylsulfoxide, N-methyl-2-pyrrolidone, etc.

"Polar protic solvent" refers to a solvent that has a hydrogen bonding ability (because it contains at least one hydrogen atom directly connected to an electronegative atom (such as O-H or N-H bond)), and non-limiting examples include methanol, water, ethanol, ammonia, acetic acid, etc.

"Optionally" or "alternatively" means that events or circumstances subsequently described may but not necessarily occur, and the description includes the occasion where the events or circumstances occur or do not occur.

During the reaction of the present invention, the reaction process is tracked by HPLC, HNMR or thin layer chromatography so as to judge whether the reaction is completed.

In the present application, "V" or "V/M" refers to the multiple of the volume of the reaction solvent relative to the mass of the starting material fed at 1 eq in the step.

In the present application, the actual amount of starting materials fed or the actual amount of products obtained is determined as follows: if the content is specified, the amount of starting materials or products = the weight of starting materials or products × the content; if the content is not specified, the amount is calculated assuming a 100% content.

### Definitions of abbreviations and key terms:

| Abbreviation code | English name | Abbreviation code | English name |
|---|---|---|---|
| X-Phos | 2-Dicyclohexylphosphino-2',4',6'-triisopropylbiphenyl | AcOH | Acetic acid |
| RuPhos | 2-Dicyclohexylphosphino-2',6'-diisopropoxy-1,1'-biphenyl | SFC | Supercritical fluid chromatography |
| dppf | 1,1'-Bis(diphenylphosphino)ferr ocene | SMB | Supercritical simulated moving bed system |
| dppp | Dianilino phenyl phosphonate | dppNap | 1,8-Bis(diphenylphosphino )naphthalene |
| dppb | 1,4-Bis(diphenylphosphino)but ane | Ligand | Ligand |
| dppbz | 1,2-Bis(diphenylphosphino)ben zene | Base | Base |
| dtbpf | 1,1'-Bis(di-tert-butylphosphino)ferrocene | Ee | Optical purity |
| Xant-phos | 4,5-Bis(diphenylphosphino)-9,9-dimethylxanthene | | |

Technical effects of the present invention are as follows.
1. The provided new route for synthesizing the intermediate represented by formula (II) has advantages of easy scale-up production, controllable quality and controllable cost; compared with the prior art routes, the route does not require chiral separation methods such as SFC, and the yield is significantly improved.
2. The synthesis process for the starting material, i.e. the intermediate represented by formula III, for synthesizing the intermediate represented by formula II has inexpensive and readily available raw materials, simple and efficient process, and great industrial prospects.

### Detailed Description of Embodiments

The present invention is further described in detail below in conjunction with examples which do not limit the present invention. Any equivalent substitutions made according to the disclosure of the present invention fall within the scope of protection of the present invention.

The structures of the compounds are determined by nuclear magnetic resonance (NMR) or (and) mass spectrometry (MS). The NMR shift (δ) is given in the unit of 10⁻⁶ (ppm). NMR is determined with NMR instruments (Bruker Avance III 400 and Bruker Avance 300), the solvent for determination is deuterated dimethylsulfoxide (DMSO-d₆), deuterated chloroform (CDCl₃), deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SC-A18 100 × 4.6 mm, 3.5 µm).

### Example 1:

### Preparation of (R)-4-(4-amino-2,6-dichlorophenoxy)-7-(methyl-d3)-2,5,6,7-tetrahydro-1H-cyclopenta[d]pyridazin-1-one (formula (II))

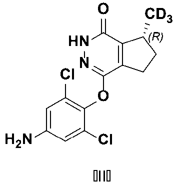

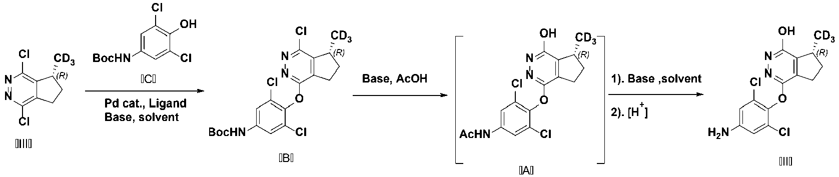

### Step 1: Preparation of tert-butyl (R)-(3,5-dichloro-4-((4-chloro-5-(methyl-d3)-6,7-dihydro-5H-cyclopenta[d]pyridazin -1-yl)oxy)phenyl)carbamate (formula (B))

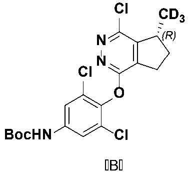

20-30V of toluene was added to a reaction kettle, the temperature was adjusted to 10°C-30°C, and potassium carbonate (3.0 eq), the intermediate represented by formula (III) (1.0 eq) and the compound represented by formula (C) (0.9 eq) were successively added. Under nitrogen protection, 2%-3% of allylpalladium dichloride and 4%-6% of 1,1-bis(ditertbutylphosphino)ferrocene were added. After the addition was completed, the mixture was warmed to 65°C-70°C and reacted for 12-24 hours with the temperature maintained constant, and then the sample was collected and delivered for detection.

The internal temperature of the reaction kettle was controlled at 10°C-30°C, 10V of water 1 was added, and the mixture was stirred for 20-40 min with the temperature maintained constant. The mixture was filtered through diatomaceous earth pad, and the kettle wall and the filter cake were washed with 2V of toluene. The filtrate was combined, the liquid separation was conducted, and the aqueous phase was discarded. Diatomaceous earth was added to the organic phase, followed by 5V of prepared aqueous citric acid solution. The internal temperature was controlled at 10°C-30°C, and the mixture was stirred for 10-30 min. The resulting mixture was filtered through diatomaceous earth pad, and the filter cake was rinsed with 1V of toluene. The liquid separation was conducted, and the aqueous phase was discarded. The organic phase was washed with 5V of water, and the liquid separation was conducted. The organic phase was concentrated to 2-3V, 5V of n-heptane was added for azeotropic distillation twice, and 10V of n-heptane was further added. The mixture was stirred for 1-3 hours at a controlled temperature of 10°C-30°C and filtered, and the kettle wall and the filter cake were rinsed with 1V of n-heptane. The filter cake was dried and detected, and the product was collected.

### Step 2: Preparation of (R)-4-(4-amino-2,6-dichlorophenoxy)-7-(methyl-d3)-2,5,6,7-tetrahydro-1H-cyclopenta[d]pyridazin-1-one (formula (II))

To reaction kettle R1 was added 10V of glacial acetic acid, and compound B (1.0 eq) and anhydrous sodium acetate (1.5 eq) were added under stirring. The mixture was warmed to 100°C-110°C, and reacted for 20-30 hours with the temperature maintained constant. Then, the reaction was cooled to 50°C-60°C, and the sample was collected for in-process control.

The resulting reaction was concentrated under reduced pressure to 2-3V at 60°C, 3V of methanol was added for azeotropic distillation twice, and 3V of methanol was further added. The feed liquid was transferred to reaction kettle R2, the temperature was controlled below 20°C-50°C, and the prepared sodium hydroxide solution was added. After the dropwise addition was completed, the temperature was controlled at 65°C-75°C, the mixture was stirred for 1-3 hours with the temperature maintained constant, and the sample was collected for in-process control.

The temperature was controlled at 65°C-75°C, and 10V of water was added dropwise to reaction kettle R2 for about 1-2 hours. After the dropwise addition was completed, the temperature was controlled at -5°C-5°C, and the mixture was stirred for 4-8 hours with the temperature maintained constant. The resulting mixture was filtered, and the kettle wall and the filter cake were washed with 2V of water. Water and citric acid were prepared in the kettle, and then filter cake and ethyl acetate were added, and the mixture was stirred for 45 minutes. The liquid separation was conducted, and mercapto silica gel was added to the organic phase. The mixture was stirred for 1 hour and filtered through diatomaceous earth pad, and the kettle wall and the filter cake were washed with ethyl acetate. The mixture was concentrated, 5V of isopropanol was added for azeotropic distillation twice, 5V of n-heptane was added for azeotropic distillation twice, and 5V of n-heptane was further added. The mixture was stirred for 1 hour and filtered, and the kettle wall and the filter cake were washed with 1V of n-heptane. To the reaction kettle were added 3V of acetonitrile, 3V of methanol and filter cake, and the mixture was warmed to 62°C and stirred until a clear solution was obtained. The temperature was controlled at 55°C-65°C, and 10V of water was added dropwise for about 0.5-2 hours. After the dropwise addition was completed, the temperature was controlled at 50°C-55°C, and the seed crystal of the intermediate represented by formula (II) was added. After maintaining the temperature for 1-2 hours, 10V of water was added dropwise for about 0.5-2 hours. After the dropwise addition was completed, the mixture was cooled to 20°C-30°C and stirred for 4-8 hours with the temperature maintained constant. The resulting mixture was filtered, and the kettle wall and the filter cake were washed with 2V of water. The filter cake was dried, and the product was collected and delivered for detection.

### Example 2: Preparation of (R)-1,4-dichloro-5-(methyl-d3)-6,7-dihydro-5H-cyclopenta[d]pyridazine (formula (III))

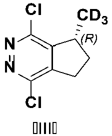

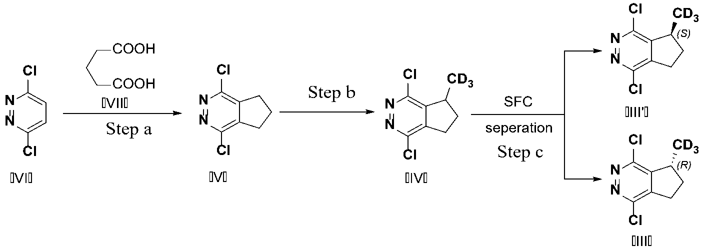

### Step a: Preparation of 1,4-dichloro-6,7-dihydro-5H-cyclopenta[d]pyridazine (formula (V))

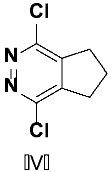

Reaction: 1200 kg of water was added to a 5000 L reaction kettle, and stirring was turned on. 12.0 kg of silver nitrate was added, the mixture was stirred until a clear solution was obtained, and 6.0 kg of concentrated sulfuric acid, 18.0 kg of glutaric acid, 60 kg of 3,6-dichloropyridazine, and 753.6 kg of 1,2-dichloroethane were slowly added. The temperature was raised to 70°C-75°C, 160.8 kg of ammonium persulphate was dissolved in 900 kg of water, and after a clear solution was obtained, the solution was added dropwise to the reaction kettle for 2-4 hours. The dropwise addition process was exothermic with carbon dioxide gas production. After the dropwise addition was completed, the mixture was reacted for 10-30 minutes with the temperature maintained constant. The reaction solution was cooled to 20°C-30°C and filtered through diatomaceous earth pad. The liquid separation was conducted, and the aqueous phase was discarded. The kettle wall and the filter cake were washed with 188.4 kg of 1,2-dichloroethane, and then the washing solution was combined with the organic phase.

1200.0 kg of water was added to a reaction kettle, and stirring was turned on. 12.0 kg of silver nitrate was added, and after a clear solution was obtained, 6.0 kg of concentrated sulfuric acid, 18.0 kg of glutaric acid and the above organic phase were added. The temperature was raised to 70°C-75°C, 160.8 kg of ammonium persulphate was dissolved in 900.0 kg of water, and after a clear solution was obtained, the mixture was added dropwise to the reaction kettle for 2-4 hours. The dropwise addition process was exothermic with carbon dioxide gas production. After the dropwise addition was completed, the mixture was reacted for 10-30 minutes with the temperature maintained constant. The reaction solution was cooled to 20°C-30°C and filtered through diatomaceous earth pad. The liquid separation was conducted, and the aqueous phase was discarded. The kettle wall and the filter cake were washed with 188.4 kg of 1,2-dichloroethane, and then the washing solution was combined with the organic phase.

1200.0 kg of water was added to a reaction kettle, and stirring was turned on. 12.0 kg of silver nitrate was added, and after a clear solution was obtained, 6.0 kg of concentrated sulfuric acid, 18.0 kg of glutaric acid and the above organic phase were added. The temperature was raised to 70°C-75°C, 160.8 kg of ammonium persulphate was dissolved in 900.0 kg of water, and after a clear solution was obtained, the mixture was added dropwise to the reaction kettle for 2-4 hours. The dropwise addition process was exothermic with carbon dioxide gas production. After the dropwise addition was completed, the mixture was reacted for 10-30 minutes with the temperature maintained constant. The reaction solution was cooled to 20°C-30°C and filtered through diatomaceous earth pad. The liquid separation was conducted, and the aqueous phase was discarded. The kettle wall and the filter cake were washed with 188.4 kg of 1,2-dichloroethane, and then the washing solution was combined with the organic phase.

1200.0 kg of water was added to a reaction kettle, and stirring was turned on. 12.0 kg of silver nitrate was added, and after a clear solution was obtained, 6.0 kg of concentrated sulfuric acid, 18.0 kg of glutaric acid and the above organic phase were added. The temperature was raised to 70°C-75°C, 160.8 kg of ammonium persulphate was dissolved in 900.0 kg of water, and after a clear solution was obtained, the mixture was added dropwise to the reaction kettle for 2-4 hours. The dropwise addition process was exothermic with carbon dioxide gas production. After the dropwise addition was completed, the mixture was reacted for 10-30 minutes with the temperature maintained constant. The reaction solution was cooled to 20°C-30°C and filtered through diatomaceous earth pad. The liquid separation was conducted, and the aqueous phase was discarded.

Post-treatment: 20.0 kg of sodium sulphite and 75.0 kg of water were prepared into a sodium sulphite solution to wash the organic phase. A sample was tested with starch-potassium iodide test paper. When the test paper showed no colour change, the mixture was stirred for another 10-20 minutes and retested. After confirming that the mixture was non-oxidizing, the liquid separation was conducted. 60.0 kg of potassium carbonate and 600.0 kg of water were prepared into a potassium carbonate solution, and after a clear solution was obtained, the resulting solution was for later use. The organic phase was concentrated until no obvious fractions appeared, and 133.2 kg of methyl tert-butyl ether was added for azeotropic distillation until no obvious fractions appeared. 222.0 kg of methyl tert-butyl ether was further added, and the mixture was stirred for 20-40 minutes. The internal temperature was controlled at 10°C-30°C, and half of the above-prepared potassium carbonate solution was added. The mixture was stirred for 20-40 minutes and then filtered through diatomaceous earth pad. The kettle wall and the filter cake were rinsed with 133.2 kg of methyl tert-butyl ether, the filtrate was allowed to stand for 5-6 hours for liquid separation, and the aqueous phase was treated as waste liquid. The organic phase was washed with the other half of the potassium carbonate aqueous solution, and allowed to stand for 5-6 hours for liquid separation, and the aqueous phase was treated as waste liquid. 18.0 kg of anhydrous magnesium sulphate was added to the organic phase, the mixture was stirred for 1-2 hours and filtered, and the kettle wall and the filter cake were washed with 44.4 kg of methyl tert-butyl ether. The organic phase was concentrated until no obvious fractions appeared. 40.5 kg of ethyl acetate was added, and the mixture was stirred at a controlled temperature of 20°C-30°C for 20-40 minutes. Two-thirds of the solution of the compound represented by formula (V) in ethyl acetate was transferred out, 30.0 kg of silica gel and 153.9 kg of n-heptane were added to the reaction kettle, and the mixture was stirred for 1-2 hours and filtered. Ethyl acetate and n-heptane were further added to the reaction kettle, and the mixture was stirred for 0.5-1 hour. The reaction kettle and the filter cake were rinsed, and the washing solution was combined with the filtrate for concentration. Another one-third of the solution of the compound represented by formula (V) in ethyl acetate was transferred in, and the above operations were repeated twice. The organic phases were combined and concentrated until no obvious fractions appeared. Methyl tert-butyl ether was added, and the mixture was stirred at a controlled temperature of 20°C-40°C for 0.5-1 hour. 4M hydrogen chloride/ethyl acetate solution was added dropwise, and after the dropwise addition was completed, n-heptane was added dropwise at a controlled temperature of 20°C-40°C for about 1-2 hours. After the dropwise addition was completed, the mixture was stirred for 0.5-1 hour, cooled to 0°C-5°C and stirred for 5-8 hours with the temperature maintained constant. The resulting mixture was filtered, and the kettle wall and the filter cake were rinsed with n-heptane. The filter cake was transferred for drying under reduced pressure, and the product was collected to obtain 18.2 kg of the hydrochloride salt of the compound represented by formula (V).

72.8 kg of purified water was added to the reaction kettle. Stirring was turned on, the internal temperature was controlled at 10°C-25°C, and then 7.3 kg of 30% sodium hydroxide solution was added. The temperature was controlled at 10°C-25°C, and 107.8 kg of methyl tert-butyl ether was added. The temperature was controlled at 10°C-25°C, the hydrochloride salt of the compound represented by formula (V) obtained above was added in portions, and the mixture was stirred for 20-40 min with the temperature maintained constant. The reaction solution was subjected to pressure filtration through a filter press. After the pressure filtration was completed, the kettle wall was rinsed with 26.9 kg of methyl tert-butyl ether, and then the filter cake was washed. The filtrate was combined and transferred into the reaction kettle. The filtrate was allowed to stand for 20-30 min for layer separation, and the organic phase was collected. 91.0 kg of purified water was added to the reaction kettle, the mixture was stirred for 20-40 min and allowed to stand for 20-30 min for liquid separation, and the organic phase was collected. The temperature was controlled at < 40°C, and the solvent was removed under reduced pressure. 81.0 kg of tetrahydrofuran was added, the temperature was controlled at < 50°C, and the solvent was removed under reduced pressure. 81.0 kg of tetrahydrofuran was added, the temperature was controlled at < 50°C, and the solvent was removed under reduced pressure. 61.9 kg of n-heptane was added, the temperature was controlled at < 50°C, and the solvent was removed under reduced pressure. 61.9 kg of n-heptane was added, the temperature was controlled at < 50°C, and the solvent was removed under reduced pressure. 61.9 kg of n-heptane was added, the temperature was controlled at 30°C-40°C, and the mixture was stirred for 1-2 hours and subjected to pressure filtration. The filter cake was washed with 12.4 kg of n-heptane, subjected to pressure filtration until no filtrate remained, and then purged with nitrogen for 4-6 hours. The filter cake was collected and dried under vacuum at 40°C for 16 hours, and then the product was collected to obtain the compound represented by formula (V).

### Step 2: 1,4-dichloro-5-(methyl-d3)-6,7-dihydro-5H-cyclopenta[d]pyridazine (the compound represented by formula (IV))

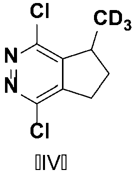

25 L of tetrahydrofuran and 2.5 kg of the compound represented by formula (V) were added to a 50 L reaction kettle, and stirring was turned on. Under nitrogen protection, the mixture was cooled to -50°C - -60°C, and 1.842 kg of lithium diisopropylamide was added dropwise with the temperature maintained constant. After the dropwise addition was completed, the mixture was stirred for 1 hour, the temperature was controlled at -50°C - -60°C, and 2.588 kg of deuterated iodomethane was added dropwise. After the dropwise addition was completed, the mixture was naturally warmed to room temperature, and reacted for 2 hours, and a sample was collected and delivered for detection.

The temperature was controlled at 0°C-10°C, 5V of 10% sodium bicarbonate aqueous solution was added with stirring, and the liquid separation was conducted. The aqueous phase was extracted once with 5V of methyl tert-butyl ether, and the organic phases were combined and washed once with 5V of water. Then, 5% wt activated carbon was added to the organic phase, and the mixture was stirred for decolourization. The resulting mixture was filtered, and the solvent was removed. 5V of n-heptane was added for azeotropic distillation twice until the volume was reduced to 1-2V, the mixture was filtered, and the filter cake was rinsed with 0.5V of isopropanol and then dried by blowing at 50°C-60°C to obtain the compound represented by formula (IV).

### Step 3: (R)-1,4-dichloro-5-(methyl-d3)-6,7-dihydro-5H-cyclopenta[d]pyridazine (the intermediate represented by formula (III))

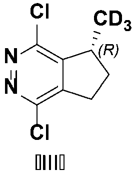

### SFC preparative conditions:

Instrument: Thar200 preparative SFC (SFC-7);
Preparation column: ChiralPak IG, 300 × 50 mm I.D., 10 µm;
Mobile phase A: CO₂, mobile phase B: methanol;
Mobile phase gradient: B 20%;
Flow rate: 200 mL/min;
Back pressure: 100 bar;
Column temperature: 38°C;
Detection wavelength: 280 nm;
Cycle time: 4.5 min;
Sample preparation: The compound was dissolved in 1200 mL of methanol/dichloromethane solution;
Sample injection: 3 mL/injection;
Post-treatment: After the preparation was completed, the resulting solution was subjected to rotary evaporation to dryness at 40°C to obtain the target compound.

### Example 3: 1,4-dichloro-5-(methyl-d3)-6,7-dihydro-5H-cyclopenta[d]pyridazine (the compound represented by formula (IV))

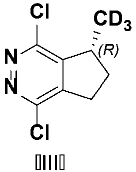

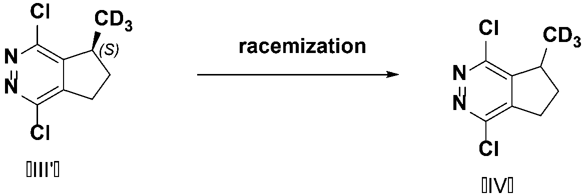

26.4 L of tetrahydrofuran and 8.74 kg of the compound represented by formula (III') were added to a 100 L reaction kettle, and the mixture was stirred for dissolution. The temperature was controlled at -50 ± 5°C, and lithium diisopropylamide (2 M, 42.5 L, 1.2 eq) was added dropwise. After the addition was complete, the mixture was reacted with the temperature maintained constant. After the reaction was completed, the temperature was controlled at 0°C-10°C, 5V of 10% sodium bicarbonate aqueous solution was added with stirring, and the liquid separation was conducted. The aqueous phase was extracted once with 5V of methyl tert-butyl ether, and the organic phases were combined and washed once with 5V of water. Then, 5% wt activated carbon was added to the organic phase, and the mixture was stirred for decolourization. The resulting mixture was filtered, and the solvent was removed. 5V of n-heptane was added for azeotropic distillation twice until the volume was reduced to 1-2V, the mixture was filtered, and the filter cake was rinsed with 0.5V of isopropanol and then dried by blowing at 50°C-60°C to obtain the compound represented by formula (IV).

### Example 4: (R)-4-(4-amino-2,6-dichlorophenoxy)-7-(methyl-d3)-2,5,6,7-tetrahydro-1H-cyclopenta[d]pyridazin-1-one (the intermediate represented by formula (II))

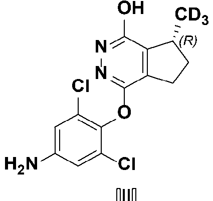

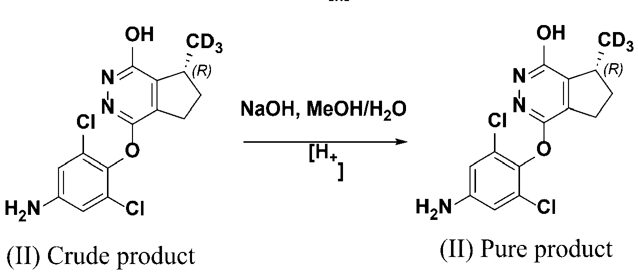

40 L of methanol, 20 L of purified water, and the crude intermediate represented by formula (II) (4.0 kg, ee = 90%) were added to a reaction kettle, and sodium hydroxide solution (5 kg of NaOH, 20 L of purified water) was added dropwise at room temperature. After the dropwise addition was completed, the mixture was warmed to reflux, the temperature was controlled at 65°C-75°C, and water was added dropwise to reaction kettle R2 for about 1-2 hours. After the dropwise addition was completed, the temperature was controlled at -5°C-5°C, and the mixture was stirred for 4-8 hours with the temperature maintained constant. The resulting mixture was filtered, and the kettle wall and the filter cake were washed with water. Water and citric acid were prepared in the kettle, and then filter cake and ethyl acetate were added, and the mixture was stirred for 45 minutes. The liquid separation was conducted, and mercapto silica gel was added to the organic phase. The mixture was stirred for 1 hour and filtered through diatomaceous earth pad, and the kettle wall and the filter cake were washed with ethyl acetate. The mixture was concentrated, isopropanol was added for azeotropic distillation twice, n-heptane was added for azeotropic distillation twice, and n-heptane was further added. The mixture was stirred for 1 hour and filtered, and the kettle wall and the filter cake were washed with n-heptane. To the reaction kettle were added acetonitrile, methanol, and filter cake, and the mixture was warmed to 62°C and stirred until a clear solution was obtained. The temperature was controlled at 55°C-65°C, and water was added dropwise for about 0.5-2 hours. After the dropwise addition was completed, the temperature was controlled at 50°C-55°C, and the seed crystal of the intermediate represented by formula (II) was added. After maintaining the temperature for 1-2 hours, water was added dropwise for about 0.5-2 hours. After the dropwise addition was completed, the mixture was cooled gradually to 20°C-30°C and stirred for 4-8 hours with the temperature maintained constant. The resulting mixture was filtered, and the kettle wall and the filter cake were washed with water. The filter cake was dried, and the product was collected to obtain the refined intermediate represented by formula (II) (yield: 88%, ee > 99.5%).

## Claims

1. A method for preparing an intermediate represented by formula (II), **characterized in that** the method comprises the following process route:

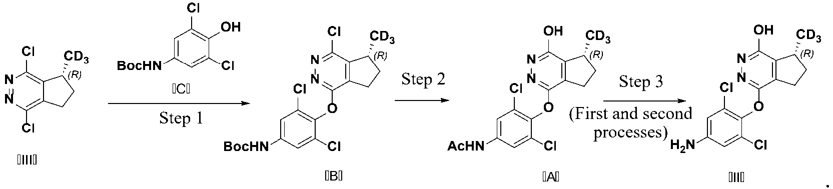

2. The preparation method according to claim 1, **characterized in that** the step 1 comprises: coupling an intermediate represented by formula (III) with a compound represented by formula (C) under nitrogen atmosphere in the presence of a palladium catalyst, a ligand, a base, and a solvent under heating or at room temperature to obtain a compound represented by formula (B).

3. The preparation method according to claim 2, **characterized in that** the palladium catalyst is selected from at least one of palladium acetate, palladium dichloride, tetrakis(triphenylphosphine)palladium, allylpalladium chloride dimer, bis(2-methylallyl)palladium chloride, (2-butenyl)chloropalladium dimer, bis(acetonitrile)palladium dichloride, tris(dibenzylideneacetone)dipalladium, bis(dibenzylideneacetone)palladium, palladium trifluoroacetate and palladium hydroxide.

4. The preparation method according to claim 2, **characterized in that** the ligand is selected from at least one of a monophosphorus ligand or a diphosphorus ligand; the monophosphorus ligand is selected from at least one of triphenylphosphine, tributylphosphine, tricyclohexylphosphine, diadamantylbutylphosphine, X-Phos, RuPhos and tri(2-furyl)phosphine; and the diphosphorus ligand is selected from at least one of dppf, dppp, dppb, dppbz, dtbpf, dppNap, Xant-phos, an oxygen-phosphorus ligand, a nitrogen-phosphorus ligand, a nitrogen-nitrogen ligand and a nitrogen-oxygen ligand.

5. The preparation method according to claim 2, **characterized in that** the base is selected from at least one of lithium carbonate, sodium carbonate, potassium carbonate, caesium carbonate, sodium bicarbonate, potassium bicarbonate, lithium acetate, sodium acetate, potassium acetate, caesium acetate, triethylamine and diisopropylethylamine.

6. The preparation method according to claim 2, **characterized in that** the solvent is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

7. The preparation method according to claim 1, **characterized in that** the reaction in the step 2 is carried out under alkaline conditions, and the base used is selected from at least one of lithium acetate, sodium acetate, potassium acetate, caesium acetate, potassium phosphate, sodium phosphate, disodium phosphate, dipotassium phosphate, sodium hydroxide, lithium hydroxide, potassium hydroxide, calcium hydroxide, caesium hydroxide (hydrate), lithium carbonate, sodium carbonate, potassium carbonate, caesium carbonate, calcium carbonate, sodium bicarbonate, potassium bicarbonate and lithium bicarbonate; and the reaction solvent is selected from at least one of toluene, benzene, N,N-dimethylacetamide, N,N-dimethylformamide, N-methylpyrrolidone, dimethylsulfoxide, 1,4-dioxane, tetrahydrofuran, 2-methyltetrahydrofuran, ethylene glycol dimethyl ether, methyl tert-butyl ether, methanol, ethanol, isopropanol, dichloroethane, dichloromethane, sulfolane, ethyl acetate, isopropyl acetate, trifluoroethanol, 1,1,1,2,2,2-hexafluoroisopropanol, acetone, butanone, methyl isobutyl ketone, acetonitrile and propanenitrile.

8. The preparation method according to claim 1, **characterized in that** the step 3 comprises a first process and a second process, the first process is carried out under alkaline conditions, and the base used is selected from at least one of lithium hydroxide, sodium hydroxide, potassium hydroxide, caesium hydroxide and a hydrate thereof, lithium carbonate, sodium carbonate, potassium carbonate, caesium carbonate, lithium acetate, sodium acetate, potassium acetate and caesium acetate; the second process is carried out under acidic conditions, and the acid used is selected from at least one of hydrochloric acid, sulfuric acid, nitric acid, formic acid, glacial acetic acid, methanesulfonic acid, p-toluenesulfonic acid and a hydrate thereof, citric acid, oxalic acid, ammonium bisulphate and sodium bisulphate; and the reaction solvent used in the step 3 is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

9. The preparation method according to claim 1, **characterized in that** the method further comprises the following purification step: 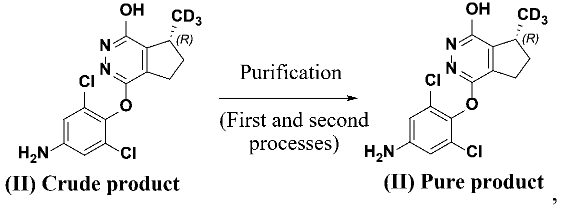 wherein the first process is carried out under alkaline conditions, and the base used is selected from at least one of lithium hydroxide, sodium hydroxide, potassium hydroxide, caesium hydroxide and a hydrate thereof, lithium carbonate, sodium carbonate, potassium carbonate, caesium carbonate, lithium acetate, sodium acetate, potassium acetate and caesium acetate; the second process is carried out under acidic conditions, and the acid used is selected from at least one of hydrochloric acid, sulfuric acid, nitric acid, formic acid, glacial acetic acid, methanesulfonic acid, p-toluenesulfonic acid and a hydrate thereof, citric acid, oxalic acid, ammonium bisulphate and sodium bisulphate; and the solvent used in the purification step is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.

10. A method for preparing an intermediate represented by formula (III), **characterized in that** the method comprises the following process route:

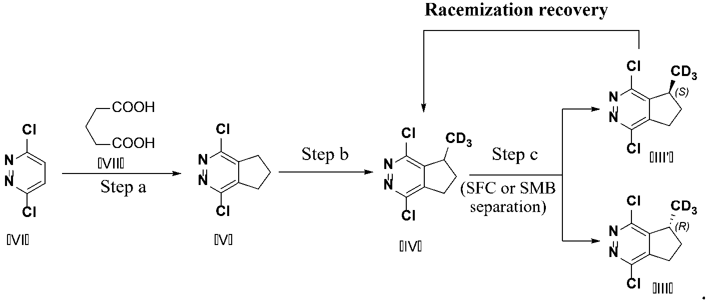

11. The preparation method according to claim 10, **characterized in that** the step a comprises: subjecting a compound represented by formula (VI) and a compound represented by formula (VII) to a Minisci reaction in the presence of a silver salt, persulphate and a solvent to obtain a compound represented by formula (V); the silver salt is selected from at least one of silver carbonate, silver nitrate, silver iodide, silver bromide, silver chloride and silver sulphate; the persulphate is selected from at least one of ammonium persulphate, sodium persulphate and potassium persulphate; and the solvent is selected from at least one of dichloromethane, trichloromethane, purified water, N,N-dimethylacetamide, N,N-dimethylformamide, N-methylpyrrolidin-2-one, dimethylsulfoxide, sulfolane, n-hexane, n-heptane, glacial acetic acid, methanol, ethanol, isopropanol, acetonitrile, propanenitrile, tetrahydrofuran and 2-methyltetrahydrofuran.

12. The preparation method according to claim 10, **characterized in that** the step b comprises: subjecting the compound represented by formula (V), after being treated with a base, to a substitution reaction with a deuterated methylating reagent to obtain a compound represented by formula (IV); the base is selected from at least one of lithium diisopropylamide, lithium hexamethyldisilazide, caesium carbonate, potassium carbonate, potassium tert-butoxide, sodium tert-butoxide, n-butyllithium and tert-butyllithium; and the deuterated methylation reagent is selected from at least one of deuterated iodomethane, deuterated dimethyl sulphate, deuterated methyl trifluoromethanesulfonate and deuterated methyl p-toluenesulfonate.

13. The preparation method according to claim 10, **characterized in that** the racemization recovery comprises: subjecting a compound represented by formula (III') to a racemization reaction in the presence of a base and a solvent to obtain the compound represented by formula (IV); the base is selected from at least one of potassium tert-butoxide, sodium tert-butoxide, tert-butyllithium, n-butyllithium, sodium ethoxide, sodium methoxide, caesium carbonate, potassium carbonate, sodium carbonate, lithium diisopropylamide, lithium hexamethyldisilazide, sodium hydroxide, potassium hydroxide, potassium phosphate, sodium phosphate, lithium hydroxide, caesium hydroxide and a hydrate thereof; and the solvent is selected from at least one of methanol, ethanol, isopropanol, ethyl acetate, isopropyl acetate, acetic acid, formic acid, methyl tert-butyl ether, acetonitrile, propanenitrile, n-butanol, dimethylsulfoxide, sulfolane, N,N-dimethylacetamide, N,N-dimethylformamide, tetrahydrofuran, 2-methyltetrahydrofuran, acetone, butanone, cyclohexane, n-heptane, benzene and toluene.

14. A compound represented by formula (B):

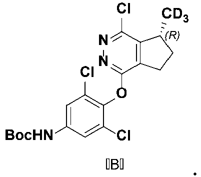

15. A method for preparing a compound represented by formula (B), **characterized in that** the method comprises the following step 1: 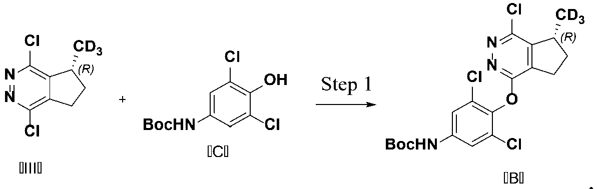
wherein the step 1 comprises: coupling the intermediate represented by formula (III) with the compound represented by formula (C) under nitrogen atmosphere in the presence of a palladium catalyst, a ligand, a base, and a solvent under heating or at room temperature to obtain the compound represented by formula (B);
the palladium catalyst is selected from at least one of palladium acetate, palladium dichloride, tetrakis(triphenylphosphine)palladium, allylpalladium chloride dimer, bis(2-methylallyl)palladium chloride, (2-butenyl)chloropalladium dimer, bis(acetonitrile)palladium dichloride, tris(dibenzylideneacetone)dipalladium, bis(dibenzylideneacetone)palladium, palladium trifluoroacetate and palladium hydroxide;
the ligand is selected from at least one of a monophosphorus ligand or a diphosphorus ligand; the monophosphorus ligand is selected from at least one of triphenylphosphine, tributylphosphine, tricyclohexylphosphine, diadamantylbutylphosphine, X-Phos, RuPhos and tri(2-furyl)phosphine; the diphosphorus ligand is selected from at least one of dppf, dppp, dppb, dppbz, dtbpf, dppNap, Xant-phos, an oxygen-phosphorus ligand, a nitrogen-phosphorus ligand, a nitrogen-nitrogen ligand and a nitrogen-oxygen ligand;
the base is selected from at least one of lithium carbonate, sodium carbonate, potassium carbonate, caesium carbonate, sodium bicarbonate, potassium bicarbonate, lithium acetate, sodium acetate, potassium acetate, caesium acetate, triethylamine and diisopropylethylamine; and
the solvent is selected from at least one of methanol, ethanol, propanol, isopropanol, tert-butanol, tetrahydrofuran, 1,4-dioxane, ethylene glycol dimethyl ether, ethylene glycol monomethyl ether, cyclopentyl methyl ether, anisole, benzene, toluene, xylene, mesitylene, chlorobenzene, dichlorobenzene, acetone, 2-butanone, 4-methyl-2-pentanone, N,N-dimethylacetamide, N,N-dimethylformamide, N-methylpyrrolidin-2-one, dimethylsulfoxide, sulfolane, n-hexane, n-heptane, glacial acetic acid and purified water.
